(19) Europäisches Patentamt
European Patent Office
Office européen des brevets

(11) EP 3 190 209 B1

(12) EUROPEAN PATENT SPECIFICATION

(45) Date of publication and mention of the grant of the patent:
**06.06.2018 Bulletin 2018/23**

(51) Int Cl.:
***C23C 18/34*** *(2006.01)* ***C23C 18/36*** *(2006.01)*
***C07C 309/15*** *(2006.01)* ***C07C 309/51*** *(2006.01)*

(21) Application number: **16150308.1**

(22) Date of filing: **06.01.2016**

# (54) 1-ACYLGUANIDINE COMPOUNDS AND THE USE OF SAID COMPOUNDS IN ELECTROLESS DEPOSITION OF NICKEL AND NICKEL ALLOY COATINGS

1-ACYLGUANIDINVERBINDUNGEN UND VERWENDUNG DIESER VERBINDUNGEN BEI DER STROMLOSEN ABSCHEIDUNG VON NICKEL UND NICKELLEGIERUNGSBESCHICHTUNGEN

COMPOSÉS 1-ACYLGUANIDINE ET UTILISATION DE CES COMPOSÉS DANS UN DÉPÔT AUTOCATALYTIQUE DE NICKEL ET DE REVÊTEMENTS D'ALLIAGE DE NICKEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.07.2017 Bulletin 2017/28**

(73) Proprietor: **Atotech Deutschland GmbH**
**10553 Berlin (DE)**

(72) Inventors:
- **Brunner, Dr. Heiko**
  **10247 Berlin (DE)**
- **Pape, Simon**
  **10713 Berlin (DE)**
- **Bejan, Dr. Iulia**
  **13156 Berlin (DE)**

(56) References cited:
**EP-A1- 0 152 601**
**EP-A1- 2 394 987**
**EP-A2- 1 281 787**
**AU-A1- 2004 248 859**
**DE-A1-102013 202 254**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Field of the Invention

The present invention relates to novel chemical compounds which are 1-acylguanidine compounds and to a method of manufacturing said compounds. Such chemical compounds are favorably applicable in an aqueous plating bath composition for the electroless deposition of nickel and nickel alloys. Therefore, the present invention further relates to an aqueous plating bath composition for the electroless deposition of nickel or nickel alloys and to a method of utilizing the aqueous plating bath composition for electrolessly depositing nickel and nickel alloys. The present invention also relates to a method of accelerating an electroless plating bath composition containing at least one of said accelerating compounds.

Nickel and nickel alloy coatings are suitable as functional coatings in aerospace, automotive, electronics, and chemical industries. The metal coatings deposited from such aqueous plating bath compositions are also useful as barrier and cap layers in semiconducting devices, printed circuit boards, IC substrates, and the like. The metal layers deposited are also suitable as an overcoat for hard disks or rigid memory disks (RMD).

### Background of the Invention

Barrier layers are used in electronic devices such as semiconducting devices, printed circuit boards, IC substrates, and the like to separate layers of different compositions, e.g., substrate layers and further layers, from each other and thereby prevent undesired diffusion between such layers of different compositions.

Another application of barrier layer materials in electronic devices is as a cap layer which is for example deposited onto copper to prevent corrosion of copper.

Rigid memory disks are used as magnetic data storage media in hard disk drives. The disks are basically composed of a substrate, made of aluminum, glass or ceramics. An overcoat is deposited onto the substrate by a vacuum deposition process or an electroless metal plating process. The overcoat may consist of various metallic, mostly non-magnetic, alloys, one of which may be a nickel/phosphorus alloy layer. The overcoat for example provides a smooth surface onto which the magnetic recording layers are deposited. Further protective layers are coated onto the recording layers.

Another application of nickel and nickel alloy deposits is the corrosion protection for various substrates.

Compositions for electroless nickel plating solutions are known in the art. For example, US Patent No. 2,762,723 teaches the use of sulfide and sulfur bearing additives in an electroless nickel plating baths for improving bath stability.

EP 0 152 601 A1 teaches an aqueous alkaline bath for the chemical deposition of copper, nickel, cobalt, and the alloys thereof. Said bath contains compounds of these metals, reducing agents, surfactants, pH adjusting agents, stabilizers, inhibitors, and complexing agents, wherein the complexing agents are polyols and/or compounds being of the biuret type. EP1281787 discloses the use of guanidine as accelerator for electroless metal plating.

### Objective of the Invention

It is an objective of the present invention to provide a plating bath composition for the deposition of nickel or a nickel alloy which has a high plating speed while stability against undesired decomposition of the plating bath composition shall not be negatively affected. As an additional objective of the present invention the plating bath composition shall have high stability also during use and during storage and shall be insensitive to concentration fluctuations of the components thereof. It is a further objective of the present invention to provide nickel and nickel alloy plating bath compositions which are suitable to generate a nickel or nickel alloy coating which has good mechanical properties and which, in particular, exhibits high compressive stress so that the corrosion resistance thereof is high. Furthermore, electroless nickel/phosphorus (Ni-P) or nickel/boron (Ni-B) or nickel/phosphorus/boron (Ni-P-B) alloys being deposited shall have a phosphorus or boron content that, in particular, does not or only to a minor extent depend on operation conditions and on concentration fluctuations of the components thereof.

### Definitions

The term "alkyl" as used herein refers to a saturated linear or branched-chain monovalent hydrocarbon radical of one to twelve carbon atoms ($C_1$-$C_{12}$), preferably of one to four carbon atoms ($C_1$-$C_4$), wherein the alkyl radical may be optionally substituted independently with one or more substituents described below. Examples of alkyl groups include, but are not limited to, methyl ($-CH_3$), ethyl ($-CH_2CH_3$), 1-propyl ($-CH_2CH_2CH_3$), 2-propyl ($-CH(CH_3)_2$), 1-butyl ($-CH_2CH_2CH_2CH_3$), 2-methyl-1-propyl ($-CH_2CH(CH_3)_2$), 2-butyl ($-CH(CH_3)CH_2CH_3$), 2-methyl-2-propyl ($-C(CH_3)_3$), 1-pentyl ($-CH_2CH_2CH_2CH_2CH_3$), 2-pentyl ($-CH(CH_3)CH_2CH_2CH_3$), 3-pentyl ($-CH(CH_2CH_3)_2$), 2-methyl-2-butyl

($-C(CH_3)_2CH_2CH_3$), 3-methyl-2-butyl ($-CH(CH_3)CH(CH_3)_2$), 3-methyl-1-butyl ($-CH_2CH_2CH(CH_3)_2$), 2-methyl-1-butyl ($-CH_2CH(CH_3)CH_2CH_3$), 1-hexyl ($-CH_2CH_2CH_2CH_2CH_2CH_3$), 2-hexyl ($-CH(CH_3)CH_2CH_2CH_2CH_3$), 3-hexyl ($-CH(CH_2CH_3)(CH_2CH_2CH_3)$), 2-methyl-2-pentyl ($-C(CH_3)_2CH_2CH_2CH_3$), 3-methyl-2-pentyl ($-CH(CH_3)CH(CH_3)CH_2CH_3$), 4-methyl-2-pentyl ($-CH(CH_3)CH_2CH(CH_3)_2$), 3-methyl-3-pentyl ($-C(CH_3)(CH_2CH_3)_2$), 2-methyl-3-pentyl ($-CH(CH_2CH_3)CH(CH_3)_2$), 2,3-dimethyl-2-butyl ($-C(CH_3)_2CH(CH_3)_2$), 3,3-dimethyl-2-butyl ($-CH(CH_3)C(CH_3)_3$, 1-heptyl, 1-octyl, and the like.

The term "alkylene" as used herein refers to a saturated linear or branched-chain divalent hydrocarbon radical of one to twelve carbon atoms ($C_1$-$C_{12}$), preferably of one to four carbon atoms ($C_1$-$C_4$), wherein the alkylene radical may be optionally substituted independently with one or more substituents described hereinafter. Examples of alkylene groups include, but are not limited to, methylene ($-CH_2-$), ethylene ($-CH_2CH_2-$), propylene ($-CH_2CH_2CH_2-$), and the like.

Alkyl and alkylene may be substituted, wherein at least one hydrogen atom thereof is substituted by any radical group like aryl, heteroaryl, OR, NR'R", COOR, CONR'R", wherein R, R', and R" are independently selected from hydrogen, alkyl, aryl and heteroaryl. Heteroaryl is a monovalent radical comprising an aromatic ring system which includes at least one of N, S, and/or O, such as pyridyl, pyrryl, thiophenyl, furanyl and the like.

"Aryl" means a monovalent aromatic hydrocarbon radical of 6-20 carbon atoms ($C_6$-$C_{20}$) derived by the removal of one hydrogen atom from a single carbon atom of a parent aromatic ring system. Aryl includes bicyclic radicals comprising an aromatic ring fused to an aromatic carbocyclic ring. Typical aryl groups include, but are not limited to, radicals derived from benzene (phenyl), substituted benzenes, naphthalene and the like. Aryl radicals are optionally substituted independently with one or more substituents described herein below.

"Arylene" means a divalent aromatic hydrocarbon radical of 6-20 carbon atoms ($C_6$-$C_{20}$) derived by the removal of two hydrogen atoms from two carbon atoms of a parent aromatic ring system. Arylene includes bicyclic radicals comprising an aromatic ring fused to an aromatic carbocyclic ring. Typical arylene groups include, but are not limited to, radicals derived from benzene (phenylene), substituted benzenes, naphthalene, and the like. Arylene groups are optionally substituted independently with one or more substituents described hereinafter.

Unless otherwise defined in the general chemical formulae recited herein, aryl and arylene may be substituted with one or more substituents, wherein at least one hydrogen atom of the aryl or arylene moiety is substituted by any radical group like alkyl, another aryl, heteroaryl, OR, NR'R", COOR, CONR'R", wherein R, R' and R" are independently selected from hydrogen, alkyl, aryl and heteroaryl.

The term "halogen" or "Hal" as used herein refers to fluorine (F), chlorine (Cl), bromine (Br), or iodine (I).

Throughout the description, the invention hereinafter will exclusively refer to electroless deposition of nickel and nickel alloys. Accordingly, the description will not refer to electrolytic plating of nickel or nickel alloys.

## Summary of the Invention

In a first aspect of the present invention, these objectives are solved by providing novel compounds which are favorably suitable to be used in an aqueous plating bath composition for the electroless deposition of nickel or nickel alloy, wherein these novel compounds are 1-acylguanidine compounds having general chemical formula I:

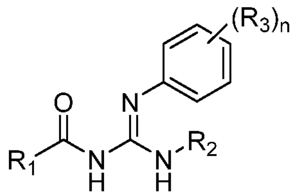

I

wherein

$R^1$ is selected from the group comprising alkyl and aryl, more preferably $R^1$ is selected from the group consisting of alkyl and aryl, wherein alkyl is unsubstituted or substituted and aryl is unsubstituted or substituted;

$R^2$ is a radical having general chemical formula $-R^4-SO_3M$;
wherein $R^4$ is selected from the group comprising alkylene and arylene, more preferably $R^4$ is selected from the group consisting of alkylene and arylene, wherein alkylene is unsubstituted or substituted and arylene is unsubstituted or substituted; and
wherein M is hydrogen or a metal atom or any other cation forming radical (such as ammonium or a quaternary amine) having a valency of one or is an $m^{th}$ fraction of a metal atom or any other cation forming radical having a valency of m, wherein m is an integer of at least 2 and which preferably is 2 or 3; if m is 2 or more, the $m^{th}$ fraction

of a metal atom or any other cation forming radical means that one metal atom or any other cation forming radical pertains to two or even more 1-acylguanidine compounds, wherein $M^{m+}$ compensates for one negative charge of the $SO_3^-$ ion each in m guanidine compounds.

$R^3$ is selected from the group comprising -Hal, $-OR^5$, and $-NR^5R^6$, more preferably $R^3$ is selected from the group consisting of -Hal, $-OR^5$, and $-NR^5R^6$, wherein $R^5$, $R^6$ are independently selected from the group comprising hydrogen, alkyl, and aryl, more preferably $R^5$, $R^6$ are selected from the group consisting of hydrogen, alkyl, and aryl, wherein alkyl is unsubstituted or substituted and aryl is unsubstituted or substituted; and

n is an integer ranging from 1 to 5; if $n > 1$, more than one $R^3$ is bonded to the phenyl ring system.

In a second aspect of the present invention, these objectives are solved by providing a method of manufacturing a compound of general chemical formula I as defined hereinbefore, said method comprising treating a 1-acylthiourea compound having general chemical formula II:

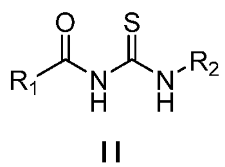

II

wherein $R^1$ and $R^2$ are as defined hereinbefore, with a primary amine compound having general chemical formula III:

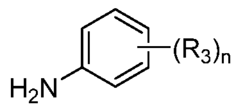

III

wherein $R^3$ and n are as defined hereinbefore.

In a third aspect of the present invention, these objectives are also solved by providing an aqueous plating bath composition for the electroless deposition of nickel or a nickel alloy, the composition comprising (i) a source of nickel ions, wherein the aqueous plating bath composition further comprises (ii) at least one accelerating compound which is a 1-acylguanidine compound having general chemical formula I which is as defined hereinbefore.

In a fourth aspect of the present invention, these objectives are also solved by providing a method of electroless depositing nickel or a nickel alloy, wherein said method comprises the following method steps: (A) providing a substrate; and (B) contacting said substrate with the aqueous plating bath composition of the invention so that a nickel or nickel alloy coating is deposited onto said substrate.

In a fifth aspect of the present invention, these objectives are also solved by providing a method of accelerating an aqueous plating bath composition for the electroless deposition of nickel or a nickel alloy, said method comprising the following method steps: (i) providing the aqueous plating bath composition; and (ii) adding at least one compound to said aqueous plating bath composition, wherein said at least one compound is a 1-acylguanidine compound having general chemical formula I as defined hereinbefore.

The novel compounds are favorably utilized in the aqueous plating bath compositions. They are compounds which accelerate deposition of nickel or nickel alloy coatings and are further advantageous relating to the stabilizing effect they exert when a nickel or nickel alloy coating is deposited. Contrary to common stabilizing species containing sulfur, especially in a low oxidation state like sulfides, thiourea for example, the 1-acylguanidine compounds of the present invention can be utilized in a wide concentration range so that concentration fluctuations of these compounds in the plating bath composition, which may occur spontaneously in the plating bath composition due to changing bath loading or the like, do not considerably influence the performance thereof to deposit nickel or nickel alloy coatings. Especially, concerning stability of the plating bath composition, mechanical stress in the coatings being deposited, and phosphorus and/or boron content in the nickel alloys being deposited, concentration fluctuations do not affect plating bath composition performance to a relevant extent. Contrary to the 1-acylguanidine compounds of the present invention, thiourea, for example, is suitable as an accelerator compound, *i.e.*, for enhancing plating speed, at a very small concentration only while this compound acts as a stabilizing compound at higher concentrations. Furthermore, thiourea and the derivatives thereof tend to release sulfur which is then incorporated into the nickel or nickel alloy coating. Such alloying of nickel with sulfur leads to increased corrosion of the coatings.

It has proved that the coatings deposited when using 1-acylguanidine compounds exhibit compression stress even if the concentration of these compounds is increased to a considerable magnitude. As conventional additives, by

contrast, lead to transition from compression stress to tensile stress if the concentration thereof in the plating bath composition is increased beyond a critical value which has shown to be relatively low compared to the novel compounds of this invention, corrosion resistance of the coatings obtained with the 1-acylguanidine compounds has shown to be much better than hitherto when using conventional additives. This is due to the fact that there is a correlation between the compression strength indicating good corrosion behavior compared to tensile stress which leads to higher aptitude towards corrosion of the coatings produced.

If plating speed is to be set high when nickel or nickel alloy coatings are to be deposited, conventional additives causing accelerated deposition will therefore inevitably lead to inferior corrosion resistance because of their presence in the plating bath composition leading to tensile stress of the coatings. The novel compounds of the invention however, yield a considerably better result because good corrosion resistance is achieved even if the plating speed is set to be high so that a sufficiently thick nickel or nickel alloy layer can be deposited quickly at short time. This advantageous effect is accompanied by the 1-acylguanidine compounds which do not adversely affect the incorporation of phosphorus and/or boron into the nickel coating, thus rendering nickel alloy plating insensitive to concentration fluctuations of the 1-acylguanidine compounds.

## Detailed Description of the Invention

The inventors have found that 1-acylguanidine compounds prove to be particular effective in accelerating plating speed of electroless nickel or nickel alloy deposition and that these compounds have the additional benefit of maintaining compressive stress in the coating being formed while not impairing stability of the plating bath composition of the present invention, when the concentration thereof is considerably increased. This is contrary to the effect conventional additives have on nickel and nickel alloy plating bath compositions, especially stabilizing compounds, which, at very low concentration, accelerate plating rate but decelerate the same at slightly raised concentrations and then deposit nickel and nickel alloy coatings which tend to exhibit tensile stress and therefore have inferior corrosion resistance. Furthermore, these compounds do not affect phosphorus content of a Ni-P alloy being formed or only influence the phosphorus content to a minor extent as the concentration of the compounds is raised.

In a preferred embodiment the present invention provides 1-acylguanidine compounds, wherein $R^1$ is $C_1$-$C_4$ alkyl or phenyl. Even more preferably, $R^1$ is unsubstituted $C_1$-$C_4$ alkyl or unsubstituted phenyl.

In an even more preferred embodiment, the present invention provides 1-acylguanidine compounds, wherein $R^1$ is phenyl, more preferably unsubstituted phenyl. Such latter compounds have the following general chemical formula IV:

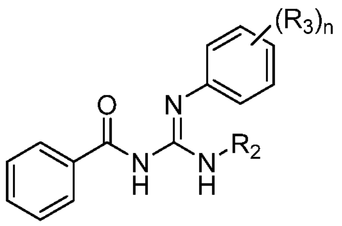

**IV**

wherein $R^2$, $R^3$, and n are as defined hereinbefore. In this embodiment phenyl is unsubstituted.

In principle, $R^1$ may also be an aromatic radical other than phenyl, naphthyl for example. In such case, it may be favorable to provide the $R^1$ radical with at least one anion forming substituent that renders the accelerating compound better soluble in an aqueous solution, such as with at least one sulfonate radical and/or with at least one carboxylic radical. Likewise, phenyl or $C_1$-$C_4$ alkyl, as mentioned before representing $R^1$, may be substituted with at least one anion forming substituent, such as at least one sulfonate radical and/or with at least one carboxylic radical.

In an even more preferred embodiment, the present invention provides 1-acylguanidine compounds, wherein $R^2$ is $-R^4-SO_3M$, wherein M is as defined hereinbefore and wherein $R^4$ is $C_1$-$C_4$ alkylene or phenylene or naphthylene, more preferably unsubstituted $C_1$-$C_4$ alkylene or unsubstituted phenylene or unsubstituted naphthylene. If $R^4$ is phenylene or naphthylene, the $-SO_3M$ radical preferably is in a 2- or 4-position (ortho- or para-position) relative to the carbon ring atom which is bonded to the guanidine nitrogen. Even more preferably, the $-SO_3M$ radical is in a 4-position relative to the carbon ring atom which is bonded to the guanidine nitrogen.

In an even more preferred embodiment, the present invention provides 1-acylguanidine compounds, wherein at least one $R^3$ is Hal, more preferably -F, or $-OR^5$ or $-NR^5R^6$, wherein $R^5$, $R^6$ are independently selected from the group comprising alkyl and aryl, more preferably alkyl, and even more preferably $C_1$-$C_4$ alkyl. In an even more preferred embodiment, at least one $R^3$ is $-OR^5$ or $-NR^5R^6$, wherein $R^5$, $R^6$ are independently alkyl or aryl, and even more preferably

at least one $R^3$ is $-OR^5$, wherein $R^5$ is alkyl or aryl. If more than one $R^3$ is present as a substituent at the phenylene ring bonded to the imine nitrogen, each one is selected independently from any other $R^3$.

Most preferably $R^3$ is $-OR^5$, wherein $R^5$ is alkyl, advantageously $C_1$-$C_4$ alkyl, even more advantageously methoxy or ethoxy.

In yet another preferred embodiment, the present invention provides 1-acylguanidine compounds, wherein $R^5$, $R^6$ are each independently a $C_1$-$C_4$ alkyl radical and wherein n is 2 or 3.

In an even more preferred embodiment, the present invention provides 1-acylguanidine compounds, wherein $R^1$ is phenyl and wherein $R^2$ is $-R^4-SO_3M$, wherein M is as defined hereinbefore and wherein $R^4$ is alkylene, advantageously $C_1$-$C_4$ alkylene, or phenylene or naphthylene, more preferably unsubstituted $C_1$-$C_4$ alkylene or unsubstituted phenylene or unsubstituted naphthylene, and wherein at least one $R^3$ is $-OR^5$, wherein $R^5$ is alkyl, advantageously $C_1$-$C_4$ alkyl.

In an even more preferred embodiment, one C atom of the phenylene ring bonded to the imine nitrogen is substituted with one $R^3$ radical only. Thus, this preferred 1-acylguanidine compound has the following general chemical formula **V**:

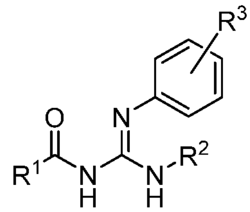

**V**

wherein:

- $R^1$ is phenyl;
- $R^2$ is alkylenesulfonate, advantageously $C_1$-$C_4$ alkylenesulfonate, more advantageously unsubstituted $C_1$-$C_4$ alkylenesulfonate, or phenylenesulfonate, more advantageously unsubstituted phenylenesulfonate, or naphthylenesulfonate, more advantageously unsubstituted naphthylenesulfonate, wherein the sulfonate radical may be the salt radical of a metal base (alkali or the like) or any other cation forming radical or may be the sulfonic acid radical; if $R^2$ is phenylenesulfonate or naphthylenesulfonate, the sulfonate radical preferably is in a 2- or 4-position relative to the carbon ring atom of the phenylene or naphthylene ring which is bonded to the guanidine nitrogen;
- $R^3$ is $-OR^5$, wherein $R^5$ is alkyl, advantageously $C_1$-$C_4$ alkyl, more advantageously unsubstituted $C_1$-$C_4$ alkyl.

In an even more preferred embodiment, the present invention provides 1-acylguanidine compounds, wherein $R^3$ is bonded to the phenylene ring in at least one of the 2- or 4-positions relative to the bonding of the phenylene ring to the imine nitrogen.

In a most preferred embodiment, the present invention provides 1-acylguanidine compounds, which are selected from:

- a salt of (E)-2-(3-benzoyl-2-(4-methoxyphenyl)guanidino)ethylsulfonate or the acid thereof;
- a salt of (E)-4-(3-benzoyl-2-(4-methoxyphenyl)guanidino)phenylsulfonate or the acid thereof;
- a salt of (E)-2-(3-benzoyl-2-(4-methoxyphenyl)guanidino)phenylsulfonate or the acid thereof; and
- a salt of (E)-8-(3-benzoyl-2-(4-methoxyphenyl)guanidino)naphthyl-2-sulfonate or the acid thereof.

In principle, the 1-acylguanidine compound may also be a salt of (E)-2-(3-benzoyl-2-(4-fluorophenyl)guanidino)phenylsulfonate or the acid thereof.

The sulfonate salt may be a metal salt such as an alkali metal salt, or an ammonium salt or quaternary amine salt or a salt of any other cation.

The 1-acylguanidine compounds of the present invention may be synthesized using a method comprising treating a thiourea compound having general chemical formula II:

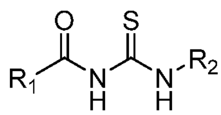

II

wherein $R^1$ and $R^2$ are as defined hereinabove, with a primary amine compound having general chemical formula III:

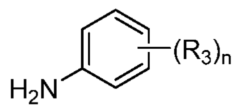

III

wherein $R^3$ and n are as defined hereinabove, in the presence of a base, like a tertiary amine compound, and a Lewis acid. The reaction may be performed using an aprotic solvent, such as acetonitrile. The Lewis acid may be selected from iron(III)chloride or another iron(III)compound, preferably inorganic or organic iron(III) salt. The reaction is preferably conducted under anhydrous conditions.

Electroless nickel plating compositions for applying nickel coatings are well known in the art and plating processes and compositions are described in numerous publications such as in US Patent Nos. 2,935,425; 3,338,726; 3,597,266; 3,915,716; and 4,780,342. Electroless plating generally comprises methods which are performed without using an external current source for reducing metal ions and depositing metal. Plating processes using external current sources are commonly described as electrolytic or galvanic plating methods. In the electroless plating solutions chemical reducing agents like hypophosphite, boranes, hydrazines, or formaldehyde are used to reduce the metal ions to their metallic form and thereby forming a deposit on the substrate.

One commonly deposited nickel alloy is Ni-P alloy. In general, Ni-P deposition compositions comprise at least three ingredients dissolved in a solvent, typically water. These ingredients are (1) at least one source of nickel ions, (2) at least one reducing agent and (3) at least one complexing agent for metal ions sufficient to prevent their precipitation in solution. A large number of suitable complexing agents for Ni-P solutions are described in the above noted publications. If hypophosphite is used as the at least one reducing agent, the deposit will contain phosphorus in addition to nickel. Similarly, if an aminoborane is employed, the deposit will contain boron in addition to nickel, as is described in US Patent No. 3,953,654.

The aqueous plating bath composition of the present invention comprises at least one source of nickel ions. The at least one source of nickel ions may be provided by the use of any soluble salt of nickel, such as nickel sulfate, nickel chloride, nickel acetate, nickel methyl sulfonate, nickel sulfamate, and mixtures thereof. The concentration of the nickel ions in the composition may vary widely and, in a preferred embodiment of the present invention, is preferably at least 0.01 mole/L, more preferably at least 0.03 mole/L and most preferably at least 0.06 mole/L. According to this preferred embodiment, the concentration of the nickel ions in the composition is at most 1 mole/L, more preferably at most 0.8 mole/L and most preferably at most 0.3 mole/L. Each combination of concentration limits indicated hereinbefore is applicable.

The aqueous plating bath composition further comprises at least one reducing agent for nickel ions to generate metallic nickel. The at least one reducing agent is preferably a chemical reducing agent. Reducing agents provide the electrons needed to reduce metal ions to their metallic form and thereby form a metal deposit on a substrate. The at least one reducing agent is preferably selected from the group comprising hypophosphite ions, borane compounds, hydrazine, and formaldehyde. In a first embodiment of the present invention, the at least one reducing agent may be a hypophosphite salt or hypophosphorous acid, more preferably a hypophosphite salt. The hypophosphite salt is supplied to the composition by any suitable source, such as sodium hypophosphite, potassium hypophosphite, ammonium hypophosphite, and nickel hypophosphite. In a second embodiment of the present invention, at least one reducing agent may be an aminoborane and/or borohydride. In a third embodiment of the present invention, at least one reducing agent may be hydrazine and/or a derivative thereof. In a fourth embodiment of the present invention, the at least one reducing agent may be formaldehyde. Two or more reducing agents may be employed as a mixture in the composition. In general, the concentration of the at least one reducing agent (total concentration of all reducing agents contained in the composition) is in molar excess of the amount sufficient to reduce the nickel ions in the composition. According to this preferred embodiment, the concentration of the at least one reducing agent (total concentration) is at least 0.01 mole/L and more preferably at least 0.1 mole/L. According to this preferred embodiment, this concentration is preferably at most 3.0 mole/L and more preferably at most 1 mole/L. Each combination of concentration limits hereinbefore indicated is applicable.

In case a hypophosphite compound is used as the reducing agent, a Ni-P alloy coating is obtained. Hypophosphite compounds provide the source of phosphorus in the deposited alloy. A borane-based compound as a reducing agent leads to a Ni-B alloy coating. And a mixture of at least one hypophosphite compound and at least one borane-based compound as the reducing agents leads to a ternary Ni-P-B alloy coating. A nitrogen-based reducing agent, such as hydrazine and derivatives thereof as well as formaldehyde, lead to nickel deposits having neither phosphorus nor boron.

The aqueous plating bath composition of the present invention may be acidic, neutral or alkaline. An acidic or an alkaline pH adjustor may be selected from a wide range of materials, such as ammonium hydroxide, sodium hydroxide, hydrochloric acid, sulfuric acid, and the like. The pH of the composition may range from about 2 to about 12. In one embodiment, the compositions are preferably acidic. More preferably, the pH of the acidic compositions ranges from 3.5 to 7, even more preferably from 3.5 to 6.5, most preferably from 3.5 to 5.5. In another embodiment, the plating compositions are preferably alkaline. In this case, the pH of the alkaline compositions more preferably ranges from 7.5 to 12, even more preferably from 8 to 10, most preferably from 8 to 9.

In a further embodiment of the present invention, the aqueous plating bath composition further comprises at least one complexing agent. A complexing agent (sometimes also referred to as chelating agent) or a mixture of complexing agents is included in the composition for nickel and nickel alloy plating. A complexing agent keeps metal ions dissolved and prevents their undesired precipitation in solution. The at least one complexing agent is preferably selected from complexing agents for nickel ions and complexing agents for alloying metal ions, more preferably complexing agents for nickel ions.

The at least one complexing agent is preferably selected from the group comprising alkyl amines, ammonia, carboxylic acids, hydroxyl carboxylic acids, aminocarboxylic acids, salts of the aforementioned complexing agents and mixtures thereof.

In a preferred embodiment of the present invention, carboxylic acids, hydroxyl carboxylic acids, aminocarboxylic acids, and the salts of the aforementioned complexing agents or mixtures thereof may be employed as the at least one complexing agent. Useful carboxylic acids include the mono-, di-, tri- and tetracarboxylic acids. The carboxylic acids may be substituted with various substituent groups, such as hydroxy or amino groups, and the acids may be introduced into the composition as their sodium, potassium or ammonium salts. Some complexing agents such as acetic acid, for example, may also act as a pH buffering agent, and the appropriate concentration of such additive components can be optimized for any composition in consideration of their dual functionality.

Examples of such carboxylic acids which are useful as complexing agents in the composition of the present invention include: monocarboxylic acids, such as acetic acid, hydroxyacetic acid (glycolic acid), aminoacetic acid (glycine), propanoic acid, 2-aminopropanoic acid (alanine); 2-hydroxypropanoic acid (lactic acid); dicarboxylic acids, such as succinic acid, aminosuccinic acid (aspartic acid), hydroxysuccinic acid (malic acid), propanedioic acid (malonic acid), tartaric acid, hexane-1,6-dicarboxylic acid (adipic acid); tricarboxylic acids, such as 2-hydroxy-1,2,3-propane tricarboxylic acid (citric acid); and tetracarboxylic acids, such as ethylene diamine tetra acetic acid (EDTA). Preferred carboxylic acids are the acids, selected from the group comprising acetic acid, aminoacetic acid, propanoic acid, 2-hydroxypropanoic acid, succinic acid, hydroxysuccinic acid, adipic acid, and 2-hydroxy-1,2,3-propane-tricarboxylic acid. In a preferred embodiment, mixtures of two or more of the above complexing/chelating agents are utilized in the composition according to the present invention.

Alkylamines may also be used as the at least one complexing agent, for example mono-, di- and trialkylamines. $C_1$-$C_3$ alkylamines, for example triethanolamine, are preferred. Ammonia may also be used as the at least one complexing agent.

In a preferred embodiment of the present invention, the concentration of the at least one complexing agent (total concentration of all complexing agents present) is preferably at least 0.01 mole/L, more preferably at least 0.1 mole/L and most preferably at least 0.2 mole/L. According to this preferred embodiment, the concentration of the at least one complexing agent is at most 3.0 mole/L, more preferably at most 1.0 mole/L and most preferably at most 0.6 mole/L. Each combination of concentration limits hereinbefore indicated is applicable.

The at least one accelerating compound of the present invention is preferably one of the preferred 1-acylguanidine compounds. Specifically, the at least one accelerating compound is selected from the group comprising those 1-acylguanidine compounds, wherein $R^1$ is unsubstituted $C_1$-$C_4$ alkyl or is unsubstituted phenyl. More preferably, the at least one accelerating compound is selected from the group comprising those 1-acylguanidine compounds, wherein $R^1$ is unsubstituted phenyl. Even more preferably, the at least one accelerating compound is selected from the group comprising those 1-acylguanidine compounds, wherein $R^4$ is unsubstituted $C_1$-$C_4$ alkylene or unsubstituted phenylene or unsubstituted naphthylene. Even more preferably, the at least one accelerating compound is selected from the group comprising those 1-acylguanidine compounds, wherein $R^3$ is bonded to the phenylene ring in at least one of the 2- or 4-positions relative to the bonding of the phenylene ring to the imine nitrogen. Even more preferably, the at least one accelerating compound is selected from the group comprising those 1-acylguanidine compounds, wherein $R^5$, $R^6$ are each $C_1$-$C_4$ alkyl and wherein n is 2 or 3. Even more preferably, the at least one accelerating compound is selected from the group comprising a salt of (E)-2-(3-benzoyl-2-(4-methoxyphenyl)guanidino)ethylsulfonate or the acid thereof; a salt of (E)-4-(3-benzoyl-2-(4-methoxyphenyl)guanidino)phenylsulfonate or the acid thereof; a salt of (E)-2-(3-benzoyl-2-(4-methoxyphenyl)guanidino)phenylsulfonate or the acid thereof; a salt of (E)-8-(3-benzoyl-2-(4-methoxyphenyl)guanidino)naphthyl-2-sulfonate or the acid thereof; and a salt of (E)-2-(3-benzoyl-2-(4-fluorophenyl)guanidino)phenylsulfonate or the acid thereof. Most preferably, the at least one accelerating compound is selected from the group consisting of (E)-2-(3-benzoyl-2-(4-methoxyphenyl)guanidino)ethylsulfonate or the acid thereof; a salt of (E)-4-(3-benzoyl-2-(4-ethoxyphenyl)guanidino)phenylsulfonate or the acid thereof; a salt of (E)-2-(3-benzoyl-2-(4-ethoxyphenyl)guanidino)phenylsulfonate or the acid thereof; and a salt of (E)-8-(3-benzoyl-2-(4-methoxyphenyl)guanidino)naphthyl-2-sulfonate or the acid thereof.

In a preferred embodiment of the present invention, the total concentration of all accelerating compounds in the aqueous plating bath composition is at least 1 μmole/L, more preferably at least 10 μmole/L. According to this preferred embodiment, the overall concentration is at most 10,000 μmole/L, more preferably at most 1,000 μmole/L. Each combination of concentration limits hereinbefore indicated is applicable.

Additionally, the plating bath composition of the present invention may include at least one stabilizing compound. Such compounds are well-known in the art. Suitable stabilizing compounds are for example sulfur compounds being at a low oxidation state, such as sulfides, namely inorganic and organic sulfides, further thiourea and the derivatives thereof, furthermore tin ions, bismuth ions, antimony ions, copper ions, and/or selenium ions. The concentration of the metal ions can vary and may range from 0.1 mg/L to 100 mg/L, more preferably from 0.1 mg/L to 50 mg/L, most preferably from 0.1 mg/L to 10 mg/L, for example. In one embodiment, the plating bath composition does not contain toxic heavy metals. In this embodiment, the composition does preferably not contain lead, cadmium, antimony, bismuth, arsenic, or mercury.

The stabilizing agents are suitable to enhance the stability of the aqueous plating bath composition of the present invention against undesired, spontaneous decomposition. Undesired, spontaneous decomposition means undesired formation of a black precipitate, undesired plate out of nickel in the bulk solution, or undesired and/or uncontrolled deposition of nickel, for example on the bottom of a plating tank or on other surfaces different from the substrate. Furthermore, if partial nickel deposition on a substrate is desired which is aimed at on catalytically active surface regions only whereas deposition of metal is undesirable on other surface regions, spontaneous decomposition of the plating bath composition may be accompanied by uncontrolled metal deposition on these other regions, too.

Stabilizing agents also impart an improved resistance of the plating bath composition against contamination with catalytic metals. Contamination with catalytic metals may be caused by metal ions dissolving from the substrate material while being in contact with the composition, or metal ions are dragged into the composition from pre-treatment or activation steps. Catalytic metals may be palladium, platinum, rhodium, ruthenium, or mixtures thereof, preferably palladium. For example, palladium is used in plating methods involving surface activation (catalyzation). Consequently, such methods cause contamination of the subsequent plating bath composition with palladium ions. An example is electroless nickel or nickel alloy plating of nonconductive substrates.

Furthermore, other materials may additionally be included in the plating bath composition of the present invention, such as pH buffers, wetting agents, additional accelerators, which are different to 1-acylguanidines, brighteners, etc. These materials are known in the art.

The plating bath composition of the present invention may further comprise at least one alloying element. In this embodiment, nickel alloy coatings containing the alloying element are deposited. The at least one alloying element may be selected from phosphorus, boron, and/or a metal which is not nickel.

Phosphorus may be comprised in the plating bath composition in the form of a hypophosphite salt and/or of hypophosphorous acid. Boron may be comprised in the plating bath composition in the form of a borane-based compound, such as aminoboranes and/or borohydrides as reducing agents, as mentioned above.

The metal which is not nickel (hereinafter abbreviated as M) may be comprised in the plating bath composition in the form of a water-soluble metal salt containing the ions of the alloying metal M. The optional alloying metal M is preferably selected from the group consisting of titanium, vanadium, chromium, manganese, zirconium, niobium, molybdenum, hafnium, tantalum, tungsten, copper, silver, gold, aluminum, iron, cobalt, palladium, ruthenium, rhodium, osmium, iridium, platinum, zinc, cadmium, gallium, indium, tin, antimony, thallium, lead, and bismuth. More preferably, the optional alloying metal M is selected from the group consisting of molybdenum, tungsten, copper, silver, gold, aluminum, zinc, and tin.

The concentration of the optional alloying metal M in the plating bath composition preferably ranges from $10^{-5}$ mole/L to 0.2 mole/L, more preferably from $10^{-4}$ mole/L to 0.2 mole/L, most preferably from $10^{-2}$ mole/L to 0.1 mole/L.

If additional metal salts or metal ions are present in the composition the respective nickel alloy Ni-M or Ni-P-M or Ni-B-M or Ni-P-B-M is obtained as a coating.

In another embodiment of the present invention, a water-soluble salt of an alloying metal M and a water-soluble salt of a second alloying metal M* are added to the composition. In this case, nickel alloy deposits comprising alloying metals M and M* are obtained.

The aqueous plating bath composition of the present invention may be manufactured by dissolving the components thereof in water, at any order, and by adjusting the pH of the composition to be in the desired range. Possibly,

the at least one accelerating compound of the present invention may preferably be pre-dissolved in a suitable co-solvent, an alcohol or an aqueous solution of a surfactant for example, prior to adding this component to water or to the aqueous solution of other components of the plating bath composition.

The present invention further relates to a method of electroless depositing nickel or a nickel alloy by contacting the substrate to be plated with the above described aqueous plating bath composition.

The substrate to be plated may be plated up to the desired thickness and deposit quantity by contacting the substrate with the composition. The composition of the present invention may be maintained over a temperature range of 20 °C to 100 °C, preferably 70 °C to 95 °C, most preferably 85 °C to 95 °C during deposition.

A deposit thickness of up to 100 μm or higher may be produced. The thickness of the nickel or Ni-P deposit preferably varies from about 1 μm to about 60 μm. The thickness depends on the technical application and can be higher or lower for some applications. For example, if the nickel or Ni-P layer is deposited to provide a corrosion resistant coating, a thickness of from about 30 μm to about 60 μm is desired, while for electronics applications a thickness of from about 1 μm to about 15 μm is applied. In the technical area of rigid memory disks, the thickness of the nickel or Ni-P deposits preferably ranges from 9 μm to 13 μm. In the technical area of semi-conductors, the thickness of the nickel or Ni-P deposits preferably ranges from 1 μm to 5 μm. Thickness of nickel or nickel alloy coatings may be measured with X-ray fluorescence (XRF) which is known in the art.

During the operation of the plating bath composition, the accelerator compounds are consumed as are the nickel ion source and reducing agents. Therefore, the amount of accelerator compounds are to be replenished. Such replenishment may be performed per metal turnover (MTO). The at least one accelerating compound of the present invention may be added as a solid or a powder or may be dissolved in a solvent prior to adding it to the plating bath composition. Preferably, the at least one accelerator compound may be dissolved in an aqueous alkaline solution prior to adding this solution to the plating bath composition.

The aqueous plating bath composition of the present invention and the methods of the present invention are suitable to provide nickel and nickel alloy coatings having an attractive bright or semi-bright appearance. Advantageously, the compressive stress of the deposited nickel or nickel alloy coatings is maintained. In contrast to accelerating compounds known in the art, the at least one accelerating compound of the present invention does not shift the internal stress of the coating to neutral or tensile stress. The advantages of nickel or nickel alloy coatings having compressive stress are high corrosion resistance and good adhesion to the substrate surface.

The above parameters of the aqueous plating bath composition of the present invention and methods of the present invention are only provided to give general guidance for practicing the invention.

A high phosphorus Ni-P alloy is herein defined as a metallic coating containing less than 91 wt.% Ni and more than 9 wt.% P, *e.g.*, 10.5 wt.%. Generally, high phosphorus Ni-P alloys contain up to 15 wt.% P. A Ni-P alloy containing more than about 10.5 wt.% phosphorus is well-known to a person skilled in the art as a high phosphorus Ni-P coating and is paramagnetic (non-magnetic) as plated. Mid phosphorus Ni-P alloys are herein defined as metallic coatings containing from 5 to 9 wt.% P. Low-phosphorus Ni-P alloys are herein defined as metallic coatings containing from 1 to 5 wt.% P.

The aqueous plating bath composition of the present invention and the methods of the present invention are suitable to provide Ni-P alloy coatings with a wide range of phosphorus content of from 1 to 15 wt.% P. The composition and the methods of the present invention are particularly suitable for depositing Ni-P alloys, *e.g.*, high phosphorus Ni-P alloys as defined above.

The at least one accelerating compound of the present invention does not change the phosphorus content of the deposited nickel alloy coatings compared to electroless Ni-P plating bath compositions not containing the at least one accelerator compound of the present invention. Thus, the at least one accelerator compound of the present invention has no negative effect on bath performance and no negative effect on coating quality.

Phosphorus content of nickel alloy coatings and thickness of nickel or nickel alloy coatings may be measured by X-ray fluorescence (XRF) which is well-known to a person skilled in the art. XRF measurements make use of the characteristic fluorescence radiation emitted from a sample (substrate, deposit) being excited with X-rays. By evaluating the wavelength and intensities and assuming a layered structure of the sample, phosphorus content and layer thickness can be calculated.

High phosphorus Ni-P alloys obtained by using the plating bath composition of the present invention contribute to generating alloys having high compressive stress. The stress values for example range from 0 to -70 $N/mm^2$, preferably from 0 to -50 $N/mm^2$, more preferably from -30 to -50 $N/mm^2$. Such deposits show high corrosion resistance and excellent adhesion to the underlying substrate onto which they are plated.

Various kinds of substrates can be metal plated with the aqueous plating bath composition of the present invention. The substrates to be metal plated can be selected from the group comprised of electrically nonconductive substrates, electrically conductive substrates, and semiconductive substrates.

The electrically nonconductive substrates to be metal plated can be selected from the group comprising glass, ceramics, and plastics, for example. Plastics can be selected from the group comprising acrylonitrile-butadiene-styrene-copolymer (ABS copolymer); polyamide; a mixture of an ABS copolymer and at least one other polymer which is different to the ABS copolymer; polycarbonate (PC); ABS/PC blends; epoxy resin; bismaleimide-triazine resin (BT); cyanate ester resin; polyimide; polyethylene terephthalate (PET); polybutylene terephthalate (PBT); polylactic acid (PLA); polypropylene (PP); and polyester, for example.

The electrically conductive substrates to be metal plated can be selected from the group comprised of metallic substrates, and conductive metal oxides, for example. The material of metallic substrates to be metal plated can be selected from the group comprised of copper, zinc, silver, gold, platinum, palladium, iron, iridium, tin, aluminum, and nickel, for example. The material of conductive metal oxides to be metal plated can be selected from indium tin oxide (ITO), antimony tin oxide (ATO) and aluminum doped zinc oxide (AZO), for example.

The semiconductive substrates to be metal plated can be selected from the group comprised of silicon, germanium, gallium, arsenic, silicon nitride, and silicon carbide for example.

## Examples

The following non-limiting examples further illustrate the present invention.

### Synthesis of Sulfonated 1-Acylguanidine Compounds

Reagents, obtained from commercial sources, were used without further purification. Reactions were carried out in 100 ml *Mettler-Toledo* EasyMax™ reactors under atmospheric conditions. Solvents were used as purchased. All products were purified by flash-chromatography (*Interchim* PuriFlash 450) on silica gel (25 μm) using ethyl acetate and methanol as eluents, or on LiChroprep RP-18 silica gel by *Merck* (40-63 μm) using water and methanol as eluents. TLC was performed on silica gel plates 60 $F_{254}$, or on RP-18 silica gel plates 60 $F_{254}$ by *Merck*. NMR spectra were measured on a *Bruker* Ascend 400 using deuterated solvents with trimethylsilane as internal standard. Chemical shifts $\delta$ are given in ppm, coupling constants *J* in Hz. Assignment of signals, as far as possible, was done using 2D-NMR spectra (HSQC). Mass spectra were obtained with *Bruker* ESI-Q-TOFmicro. XRF-measurements were done with a Fischer XDV-SDD instrument.

Synthesis of benzoylguanidines (General Method Instruction AAV 1)

The manufacture of benzoylguanidines was carried out following instructions which were modified *vis-à-vis* those which are published in: S.Cunha, B.R.de Lima, A.R.de Souza, Tetrahedron Lett. 2002, 43, 49-52.

Thiourea (5 mmole) in 30 ml acetonitrile was furnished in a 100 ml EasyMax reactor being equipped with a thermometer located therein, a reflux condenser, and a magnetic stirrer. Thereafter, the respective amine (5 mmole, 1 eq.) and triethyl amine (20 mmole, 4 eq.) were added successively. The mixture was cooled to 0°C (Tr) within 10 min. Then, anhydrous iron(III)chlorine (5 mmole, 1 eq.) was added. After heat ceased to evolve, the mixture was within 30 min set to a temperature of 40 °C (Tr). Reaction progress was monitored using TLC. After the reaction was complete, the reaction mixture was transferred to a round bottom flask, provided with activated carbon, and rotated at a rotating evaporator using a water bath at 900 mbar and 45 °C (temperature of the water bath) for a few minutes. Then the mixture was suctioned over Celite and washed with acetone. The filtrate was concentrated at the rotating evaporator. The brown suspension obtained was provided with diethyl ether in order to precipitate the triethyl amine hydrochloride which had formed. The product was suctioned over a G4 frit and washed with diethyl ether and tetrahydrofuran. The solvent was separated using the rotating evaporator. Further purification was performed using column chromatography with RP-18 silica gel.

Optimized synthesis of benzoylguandines (General Method Instruction AAV 2)

Thiourea (5 mmole) in 30 ml acetonitrile was furnished in a 100 ml EasyMax reactor being equipped with a thermometer located therein, a reflux condenser, and a magnetic stirrer. Thereafter, the respective amine (5 mmole, 1 eq.) and N,N,N',N'-tetramethylnaphthalene-1,8-diamine (10 mmole, 2 eq.) were added successively. The temperature of the mixture was within 10 min set to 40°C (Tr). Then, anhydrous iron(III)chloride (5 mmole, 1 eq.) was added. Reaction progress was monitored using TLC. After the reaction was complete, the reaction mixture was transferred to a round bottom flask, provided with activated carbon, and rotated at a rotating evaporator using a water bath at 900 mbar and 45 °C (temperature of the water bath) for a few minutes. Then the mixture was suctioned over Celite and washed with acetone. The filtrate was concentrated at the rotating evaporator. Further purification was performed using column chromatography with RP-18 silica gel.

Synthesis Example A: (E)-4-(3-benzoyl-2-(4-methoxyphenyl)guanidino)phenyl sodium sulfonate

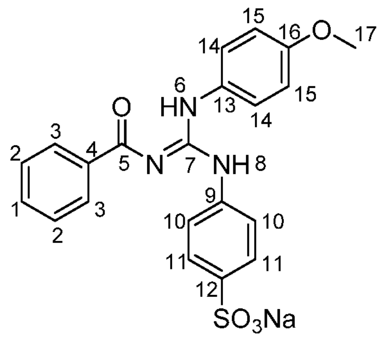

4-(3-benzoylthioureido)phenyl sodium sulfonate (1.79 g, 5 mmole) were reacted with 4-methoxyaniline (0.62 g, 5 mmole, 1 eq.), triethyl amine (2.02 g, 2.8 ml, 20 mmole, 4 eq.), and iron(III)chloride (0.81 g, 5 mmole, 1 eq.) in 30 ml DMF (dimethylformamide) according to General Method Instruction AAV 1. The reaction was terminated 22 hours after the addition of the iron(III)chloride. The reaction mixture was suctioned over Celite and purged with a small quantity of DMF and acetone. The filtrate was concentrated using the rotating evaporator to yield a dry product, which was thereafter suspended in 150 ml water and subsequently again suctioned over a G3 frit with Celite. The water was removed at the rotating evaporator. Purification of the crude product was performed with column chromatography using RP-18 silica gel (water-methanol 100:0 > 8:2 > 6:4 > 1:1). Since part of the product was present as mixed fractions, chromatographic purification had to be repeated. The product (1.32 g, 2.96 mmole, 59 %) was obtained in the form of a yellowish solid. $R_f$ (RP-18; water-methanol 7:3) = 0.15; $^1$H-NMR (400 MHz, DMSO-$d_6$, ppm): $\delta$ 3.74 (s, 3H, H-17), 6.98 (d, $^3J$ = 8.3 Hz, 2H, H-15), 7.19-7.39 (m, 4H, H-10, H-14), 7.50-7.71 (m, 5H, H-1, H-2, H-11), 7.85 (d, $^3J$ = 7.7 Hz, 2H, H-3), 11.50 (s, 2H, H-6, H-8); $^{13}$C-NMR (100 MHz, DMSO-$d_6$, ppm): $\delta$ 55.4 (C-17), 114.8 (C-15), 122.6 (C-10), 125.2 (C-14), 126.7 (C-11), 128.6 (C-3), 128.8 (C-2), 133.5 (C-1), 158.1 (C-7); HRMS (ESI): calculated for $C_{21}H_{20}N_3O_5S$ [M+H]$^+$: 426.1118, measured: 426.1102.

Synthesis Example B: (E)-2-(3-benzoyl-2-(4-methoxyphenyl)guanidino)ethyl sodium sulfonate

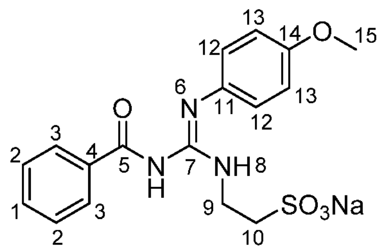

N-((4-methoxyphenyl)carbamothioyl)benzamide (1.43 g, 5 mmole) was reacted with 2-aminoethane sodium sulfonate (0.74 g, 5 mmole, 1 eq.), triethyl amine (2.02 g, 2.8 ml, 20 mmole, 4 eq.), and iron(III)chloride (0.81 g, 5 mmole, 1 eq.) according to General Method Instruction AAV 1. The reaction was terminated 66 hours after the addition of the iron(III)chlorine. The reaction mixture was suctioned over Celite and purged with a small quantity of DMF and acetone. The filtrate was concentrated using the rotating evaporator to yield a dry product, which was thereafter suspended in 150 ml water and subsequently again suctioned over a G3 frit with Celite. The water was removed at the rotating evaporator. Purification of the crude product was performed with column chromatography using RP-18 silica gel (water-methanol 95:5 > 9:1 > 85:15 > 6:4 > 1:1). The product (1.14 g, 2.86 mmole, 57 %) was obtained in the form of a yellowish solid. $R_f$ (RP-18; water-methanol 1:1) = 0.36; $^1$H-NMR (300 MHz, DMSO-$d_6$, ppm): $\delta$ 2.77 (s, 2H, H-10), 3.66-3.75 (m, 2H, H-9), 3.77 (s, 3H, H-15), 6.97 (d, $^3J$ = 8.3 Hz, 2H, H-13), 7.20-7.57 (m, 5H, H-1, H-2, H-12), 8.12 (s, 2H, H-3), 8.90 (s, 1H, NH), 11.74 (s, 1H, NH); HRMS (ESI): calculated for $C_{17}H_{20}N_3O_5S$ [M+H]$^+$: 378.1118, measured: 378.1103.

Synthesis Example C: (E)-2-(3-benzoyl-2-(4-methoxphenyl)guanidino)phenyl sodium sulfonate

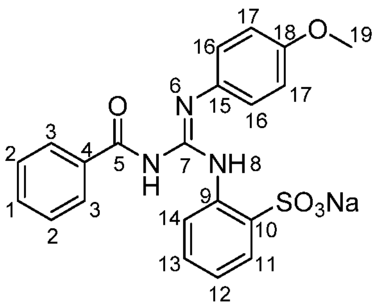

2-(3-benzoylthioureido)phenyl sodium sulfonate (7.17 g, 20 mmole) was reacted with 4-methoxyaniline (2.46 g, 20 mmole, 1 eq.), N,N,N',N'-tetramethylnaphthalene-1,8-diamine (8,57 g, 40 mmole, 2 eq.), and iron(III)chloride (3.24 g, 20 mmole, 1 eq.) in 90 ml DMF according to General Method Instruction AAV 2. The reaction was terminated 66 hours after the addition of the iron(III)chloride. Purification of the crude product was performed with column chromatography using silica gel (ethylacetate-methanol 100:0 > 95:5 > 8:2 > 6:4 > 1:1). The product (5.76 g, 12.87 mmole, 64 %) was obtained in the form of a yellowish solid. $R_f$ (ethylacetate-methanol 4:1) = 0.44; $^1$H-NMR (400 MHz, DMSO-$d_6$, ppm): $\delta$ 3.79 (s, 3H, C-19), 6.98 (d, $^3J$ = 7.8 Hz, 2H, H-17), 7.09 (t, $^3J$ = 7.6 Hz, 1H, H-12), 7.36-7.46 (m, 5H, H-2, H-13, H-16), 7.50 (t, $^3J$ = 7.0 Hz, 1H, H-1) 7.67 (d, $^3J$ = 7.7 Hz, 1 H, H-14), 8.05-8.16 (m, 3H, H-3, H-11), 9.93 (s, 1H, NH), 11.88 (s, 1H, NH); $^{13}$C-NMR (100 MHz, DMSO-$d_6$, ppm): $\delta$ 55.3 (C-19), 114.7 (C-17), 122.8 (C-12), 124.5 (C-11), 126.5 (C-14), 126.7 (C-16), 128.0 (C-2), 128.2, 128.8 (C-3), 128.9 (C-13), 131.3 (C-1), 134.6, 137.4, 138.3, 156.4, 157.6, 176.1 (C-5); HRMS (ESI): calculated for $C_{21}H_{20}N_3O_5S$ $[M+H]^+$: 426.1118, measured: 426.1112.

Synthesis Example D: (E)-2-(3-benzoyl-2-(4-fluorophenyl)guanidino)phenyl cesium sulfonate

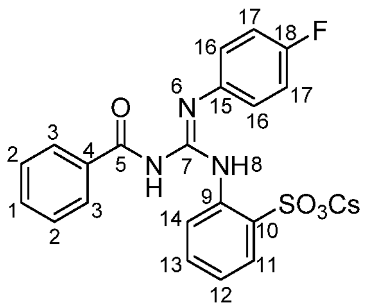

2-(3-benzoylthioureido)phenyl sodium sulfonate (2.34 g, 5 mmole) was reacted with 4-fluoraniline (0.57 g, 5 mmole, 1 eq.), N,N,N',N'-tetramethylnaphthalene-1,8-diamine (2.19 g, 10 mmole, 2 eq.), and iron(III)chloride (0.81 g, 5 mmole, 1 eq.) in 30 ml DMF according to General Method Instruction AAV 2. The reaction was terminated 68 hours after the addition of the iron(III)chloride. Purification of the crude product was performed with column chromatography using silica gel (ethylacetate-methanol 100:0 > 8:2). The product (2.22 g, 4.07 mmole, 81 %) was obtained in the form of a light brown solid. $R_f$ (ethylacetate-methanol 8:2) = 0.53; $^1$H-NMR (400 MHz, DMSO-$d_6$, ppm): $\delta$ 7.05-7.33 (m, 4H, H-12, H-13, H-17), 7.33-7.51 (m, 3H, H-14, H-16), 7.51-7.63 (m, 2H, H-2), 7.64-7.78 (m, 2H, H-1, H-11), 7.83-8.04 (m, 2H, H-3); $^{13}$C-NMR (100 MHz, DMSO-$d_6$, ppm): $\delta$ 115.6 (d, $^2J$(C,F) = 22.9 Hz, C-17), 124.0 (C-14), 125.4 (d, $^3J$(C,F) = 7.4 Hz, C-16), 126.1 (C-12), 126.8 (C-11), 128.4 (C-3), 128.5 (C-2), 129.4 (C-13), 133.3 (C-1), 160.0 (d, $^1J$(C,F) = 244.2 Hz, C-18).

Synthesis Example E: (E)-8-(3-benzoyl-2-(4-methoxyphenyl)guanidino)naphthyl-2-sulfonic acid

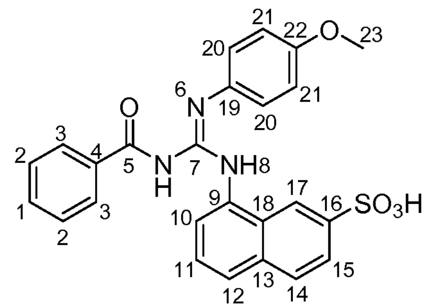

8-(3-benzoylthioureido)naphthyl-2-cesiumsulfonate (7.78 g, 15 mmole) was reacted with 4-methoxyaniline (1.85 g, 15 mmole, 1 eq.), N,N,N',N'-tetramethylnaphthalene-1,8-diamine (6.43 g, 30 mmole, 2 eq.), and iron(III)chloride (2.43 g, 15 mmole, 1 eq.) in 90 ml DMF according to General Method Instruction AAV 2. The reaction was terminated 69 hours after the addition of the iron(III)chloride. During the purification it was realized that the activated carbon retained considerable amounts of the product. Accordingly, the activated carbon was repeatedly washed with methanol. Purification of the crude product with column chromatography using silica gel (ethylacetate-methanol 100:0 > 95:5 > 8:2 > 6:4 > 1:1) was not suitable. Mixed fractions were repeatedly obtained in two passes and the product was eluted in a great number of fractions in large dilution. The combined mixed fractions were dissolved in dichloromethane and added on a short column with acidic aluminium oxide (Sigma-Aldrich, 150 nm). Then, the product was washed using dichloromethane first and subsequently using ethyl acetate. Thereafter, the product was eluted using a gradient of ethyl acetate-methanol from 10:1 > 1:1 > methanol > methanol plus cesium hydroxide. The strongly alkaline product solution was set to pH 4 using diluted hydrochloric acid in order to yield the sulfonic acid. The free acid is poorly water-soluble, precipitates and can be separated by suctioning. The product was washed two times with cold water. The product (1.33 g, 2.79 mmole, 19 %) was obtained in the form of a purple solid. $R_f$ (ethyl acetate-methanol 2:1) = 0.65; $^1$H-NMR (400 MHz, methanol-$d_6$, ppm): $\delta$ 3.76 (s, 3H, C-23), 6.89 (d, $^3J$ = 8.7 Hz, 2H, H-21), 7.31 (t, $^3J$ = 7.6 Hz, 2H, H-2), 7.37 (d, $^3J$ = 8.5 Hz, 2H, H-20), 7.43 (t, $^3J$ = 7.3 Hz, 1H, H-1), 7.59 (d, $^3J$ = 4.9 Hz, 2H, H-10, H-12), 7.83-7.91 (m, 3H, H-3, H-11), 7.96 (q, $^3J$ = 8.6 Hz, $^3J$ = 7.3 Hz, 2H), 8.63 (s, 1H, H-17); $^{13}$C-NMR (100 MHz, methanol-$d_4$, ppm): $\delta$ 55.89 (C-23), 115.23 (C-21), 121.65 (C-17), 124.69 (C-15), 126.13 (C-10), 127.62 (C-20), 128.11 (C-11), 128.33 (C-12), 128.98 (C-2), 129.93 (C-3, C-14), 130.32, 130.88, 132.74 (C-1), 136.36, 138.21, 144.00, 159.32; HRMS (ESI): calculated for $C_{25}H_{21}N_3O_5NaS$ $[M+H]^+$: 498.1100, found: 498.1066.

**Use of the 1-acylguanidine compounds of the present invention in an aqueous plating bath composition of the present invention**

An aqueous plating bath composition for the deposition of a Ni-P coating was made up of the following components:

| | |
|---|---|
| Nickel sulfate hexahydrate | 26.3 g/l |
| Lactic acid (90%) (complexing agent/chelating agent) | 14.4 g/l |
| Malic acid (complexing agent/chelating agent) | 10.0 g/l |
| Succinic acid (complexing agent/chelating agent) | 6.5 g/l |
| Sodium hypophosphite monohydrate (reducing agent) | 31.8 g/l |

Various 1-acylguanidine compounds were added to this stock solution at various concentrations to yield the aqueous plating bath composition of the present invention (see Table 2). Concentrations of these accelerator compounds in the nickel plating bath compositions were as outlined in Table 2. For comparison, one batch of the nickel plating bath composition was used without any accelerator (Table 3: *'blank'*). Another batch of the nickel plating bath composition was used with biguanidine (biuret) as another Comparative Example (Table 3).

Make-up of the plating bath compositions was performed by dissolving the respective components in water to yield the stock solution and by adding the respective accelerating compound. A plating bath container having 1 L volume was filled up with the composition and heated to 88.0 °C. The electroless nickel plating bath had a pH value of 4.8.

Steel plates were then used as substrates for deposition of Ni-P alloy coatings. The substrates were pre-treated as summarized in Table 1 in order to clean and twice zincate the substrate surface prior to nickel deposition. Uniclean® 155, Nonacid® 701 were provided by Atotech Deutschland GmbH.

Afterwards, the substrates were immersed into the respective Ni-P plating bath composition as described hereinabove. Bath loading was 0.9 $dm^2/L$ corresponding to one steel panel per bath volume. Deposition time was 120

minutes.

The deposited Ni-P alloy coatings completely covered the substrate surface. No skip plating was obtained. The deposited coatings had uniform thickness, adhered well to the substrate surface and showed good appearance with technical brightness and a typical gray color.

The phosphorus content and deposit thickness were measured at 5 points of each substrate by XRF using the XRF instrument Fischerscope XDV-SDD (Helmut Fischer GmbH, Germany). Thickness was additionally checked by using a micrometer probe (Mitutoyo, Absolute Digimatic ID S 112 XB; results shown in brackets in Tables 2, 3). The deposition rate was calculated by using the deposition time and the measured deposit thickness. Results are summarized in Tables 2, 3.

Mechanical stress in the Ni-P coatings was measured using a stress-strip finger. The test strips were made of copper and had spring like properties. After plating, as described hereinabove, the test strip was mounted on the Testing Stand (Deposit stress analyzer Model No. 683 of Specialty Testing & Development Co., York, PA, USA) which measured the distance which the test strip legs had spread after plating. The distance U is included in the following formula which allows for the deposit stress to be calculated.

$$Stress = U / 3 * T * K,$$

wherein U is the number of increments spread, T is the deposit thickness and K is the strip calibration constant.

Deposit thickness T was determined by XRF and using the micrometer probe of Mitutoyo as described hereinabove.

Each lot of test strips manufactured will respond with slight differences when used for deposit stress test. This degree of difference was determined by the supplier when each lot of test strips was calibrated. The value for K was supplied with each lot of test strips provided by Specialty Testing & Development Co.

Stress was also determined to be of compressive or tensile nature. If the test strip legs were spread outward on the side that has been plated, the deposit stress was tensile in nature. If the test strip legs were spread inward on the side that has been plated, the deposit stress was compressive in nature. The stress of the deposited Ni-P alloy coating was compressive. Results are summarized in Tables 2, 3.

The results show, that plating rate is significantly increased, when using the accelerating 1-acylguanidine compounds of the present invention. It is also shown that an increase in concentration of these compounds only slightly decreases the tendency to present compressive stress in the Ni-P coatings or to evolve tensile stress therein. Only setting the concentration of the accelerating compounds at a very large value (1000 μmol/L) may lead to neutral or tensile stress in some instances. But such shift to neutral or tensile stress does not take place for all such compounds. Furthermore, modifying the concentration of these compounds influences the phosphorus content in the Ni-P alloy coating to a minor extent only.

By contrast, biguanidine hydrochloride does not accelerate the plating speed significantly. The same behavior shows up for the blank sample (Table 3).

Table 1: Pre-Treatment of Substrates

| Pre-treatment step | Product | Concentration | Temp. (°C) | Time |
|---|---|---|---|---|
| Soak cleaner | Uniclean 155 | 60 g/l | 70 | 5 min |
| Rinse | Water | - | - | 30 s |
| Electro-cleaner | Nonacid 701 | 150 g/l | 50°C, 10 A/dm$^2$ | 1 min |
| Rinse | Water | - | - | 30 s |
| Activate | HCl (37% solution) | 1/1 (v/v) | RT | 30 s |
| Rinse | Water | - | - | 30 s |

Table 3: Results, Comparative Examples

| | | | | |
|---|---|---|---|---|
| Blank | | | | |
| | 14 (14.2) | -16 | 10.8 | |
| Comparison Example | | | | |
| 10 | - | compressive | - | 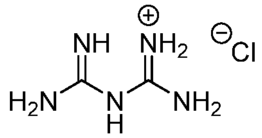 |
| 100 | 13 | -11.5 | - | |
| 1000 | 13 | -2.3 | - | |

Table 2: Results, Examples of the Present Invention

| c (μmol/L) | Plating Rate [μm/h] (XRF) | Stress [N/mm$^2$] | P Content [%] | |
|---|---|---|---|---|
| Example A | | | | 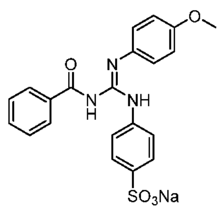 |
| 10 | 13 (15.5) | -15 | 10.3 | |
| 100 | 18 (15.8) | -8 | 10.2 | |
| 1000 | 19 (17) | -4 | 9.6 | |
| Example B | | | | 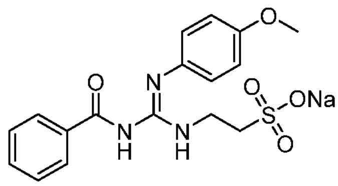 |
| 10 | - | compressive | - | |
| 100 | 18 | -2 | - | |
| 1000 | 19 | 9 | - | |
| Example C | | | | 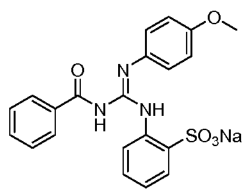 |
| 10 | 15 (15.2) | -14 | 10.7 | |
| 100 | 16 (15.7) | -14 | 10.5 | |
| 1000 | 17 (17.2) | -7 | 10.1 | |
| Example D | | | | 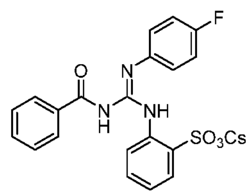 |
| 10 | 13 (13.9) | -13 | 10.5 | |
| 100 | 13 (11.9) | -9 | 9.9 | |
| 1000 | 15 (12.3) | -6 | 11 | |
| Example E | | | | 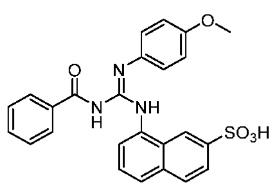 |
| 10 | 13 (14.6) | -12 | 9.9 | |
| 100 | 17 (17.3) | -5 | 9.4 | |
| 1000 | 16 (16.4) | 5 | 9.7 | |

## Claims

1. A compound which is an 1-acylguanidine compound having general chemical formula I:

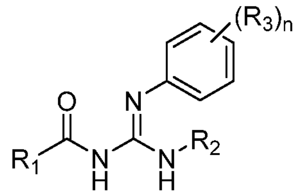

I

wherein

$R^1$ is selected from the group comprising alkyl and aryl, wherein alkyl is unsubstituted or substituted and aryl is unsubstituted or substituted;
$R^2$ is a radical having general chemical formula $-R^4-SO_3M$, wherein $R^4$ is selected from the group comprising alkylene and arylene, wherein alkylene is unsubstituted or substituted and arylene is unsubstituted or substituted; wherein M is hydrogen or a metal atom or any other cation forming radical having a valency of one or is an $m^{th}$ fraction of a metal atom or any other cation forming radical having a valency of m, wherein m is 2 or 3;
$R^3$ is selected from the group comprising -Hal, $-OR^5$, and $-NR^5R^6$, wherein $R^5$, $R^6$ are independently selected from the group comprising hydrogen, alkyl and aryl, wherein alkyl is unsubstituted or substituted and aryl is unsubstituted or substituted; and
n is an integer ranging from 1 to 5.

**2.** The compound of claim 1, wherein $R^1$ is unsubstituted $C_1$-$C_4$ alkyl or unsubstituted phenyl.

**3.** The compound of any one of the preceding claims, wherein $R^1$ is unsubstituted phenyl.

**4.** The compound of any one of the preceding claims, wherein $R^4$ is unsubstituted $C_1$-$C_4$ alkylene or unsubstituted phenylene or unsubstituted naphthylene.

**5.** The compound of any one of the preceding claims, wherein $R^3$ is bonded to the phenyl ring in at least one of the 2- or 4-positions relative to the bonding of the phenyl ring to the guanidine-N.

**6.** The compound of any one of the preceding claims, wherein $R^5$, $R^6$ are each independently $C_1$-$C_4$ alkyl and wherein n is 2 or 3.

**7.** The compound of any one of the preceding claims, which is selected from

- a salt of (E)-2-(3-benzoyl-2-(4-methoxyphenyl)guanidino)ethylsulfonate or the acid thereof;
- a salt of (E)-4-(3-benzoyl-2-(4-methoxyphenyl)guanidino)phenylsulfonate or the acid thereof;
- a salt of (E)-2-(3-benzoyl-2-(4-methoxyphenyl)guanidino)phenylsulfonate or the acid thereof;
- a salt of (E)-8-(3-benzoyl-2-(4-methoxyphenyl)guanidino)naphthyl-2-sulfonate or the acid thereof;
- a salt of (E)-2-(3-benzoyl-2-(4-fluorophenyl)guanidino)phenylsulfonate or the acid thereof.

**8.** A method of manufacturing a compound of general chemical formula I as defined in any one of claims 1 to 7, said method comprising treating a thiourea compound having general chemical formula II:

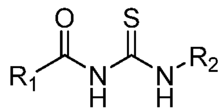

II

wherein $R^1$ and $R^2$ are as defined in claim 1,
with a primary amine compound having general chemical formula III:

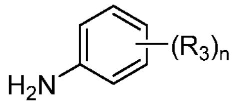

III

wherein $R^3$ and n are as defined in claim 1.

**9.** An aqueous plating bath composition for electroless deposition of nickel or a nickel alloy, the composition comprising:

(i) a source of nickel ions and at least one reducing agent for nickel ions,wherein the aqueous plating bath composition further comprises
(ii) at least one accelerating compound which is a 1-acylguanidine compound according to any one of claims 1 to 7.

**10.** The aqueous plating bath composition according to claim 9, wherein the concentration of all accelerating compounds in the aqueous plating bath composition is from 1 μmol/L to 10,000 μmol/L, preferably from 10 μmol/L to 1,000 μmol/L.

**11.** The aqueous plating bath composition according to any one of claims 9 and 10, wherein the aqueous plating bath composition further comprises at least one complexing agent.

**12.** The aqueous plating bath composition according to claim 9 to 11, wherein the at least one reducing agent is selected from the group comprising hypophosphite ions, borane compounds, hydrazine, and formaldehyde.

**13.** A method of electroless depositing nickel or a nickel alloy, said method comprising the following method steps:

(A) Providing a substrate; and
(B) Contacting said substrate with an aqueous plating bath composition according to any one of claims 9 to 11 so that a nickel or nickel alloy coating is deposited onto said substrate.

**14.** A method of accelerating an aqueous plating bath composition for the electroless deposition of nickel or a nickel alloy, said method comprising the following method steps:

(i) Providing said aqueous plating bath composition comprising a source of nickel ions and at least one reducing agent for nickel ions; and
(ii) Adding at least one compound to said aqueous plating bath composition, wherein said at least one compound is a 1-acylguanidine compound having general chemical formula I:

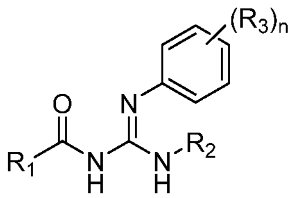

I

wherein

$R^1$ is selected from the group comprising alkyl and aryl, wherein alkyl is unsubstituted or substituted and aryl is substituted or unsubstituted;
$R^2$ is a radical having general chemical formula $-R^4-SO_3M$, wherein $R^4$ is selected from the group comprising alkylene and arylene, wherein alkylene is unsubstituted or substituted and arylene is unsubstituted or substituted;
wherein M is hydrogen or a metal atom or any other cation forming radical having a valency of one or is an $m^{th}$ fraction of a metal atom or any other cation forming radical having a valency of m, wherein m is 2 or 3;
$R^3$ is selected from the group comprising -Hal, $-OR^5$, and $-NR^5R^6$, wherein $R^5$, $R^6$ are radicals independently selected from the group comprising hydrogen, alkyl and aryl, wherein alkyl is unsubstituted or substituted

and aryl is unsubstituted or substituted; and
n is an integer ranging from 1 to 5.

## Patentansprüche

**1.** Verbindung, die eine 1-Acylguanidinverbindung der allgemeinen chemischen Formel I ist:

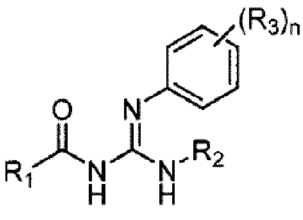

I

wobei

$R^1$ ausgewählt ist aus der Gruppe umfassend Alkyl und Aryl, wobei Alkyl unsubstituiert oder substituiert ist und Aryl unsubstituiert oder substituiert ist;
$R^2$ ein Rest mit der allgemeinen chemischen Formel $-R^4-SO_3M$ ist, wobei $R^4$ ausgewählt ist aus der Gruppe umfassend Alkylen und Arylen, wobei Alkylen unsubstituiert oder substituiert ist und Arylen unsubstituiert oder substituiert ist;
wobei M Wasserstoff oder ein Metallatom oder ein anderer kationenbildender Rest mit einer Valenz von eins ist oder ein m-ter Bruchteil eines Metallatoms oder eines anderen kationenbildenden Rests mit einer Valenz von m ist, wobei m 2 oder 3 ist;
$R^3$ ausgewählt ist aus der Gruppe umfassend -Hal, $-OR^5$ und $-NR^5R^6$, wobei $R^5$, $R^6$ unabhängig ausgewählt sind aus der Gruppe umfassend Wasserstoff, Alkyl und Aryl, wobei Alkyl unsubstituiert oder substituiert ist und Aryl unsubstituiert oder substituiert ist; und
n eine ganze Zahl in dem Bereich von 1 bis 5 ist.

**2.** Verbindung gemäß Anspruch 1, wobei $R^1$ unsubstituiertes $C_1$-$C_4$-Alkyl oder unsubstituiertes Phenyl ist.

**3.** Verbindung gemäß einem der vorstehenden Ansprüche, wobei $R^1$ unsubstituiertes Phenyl ist.

**4.** Verbindung gemäß einem der vorstehenden Ansprüche, wobei $R^4$ unsubstituiertes $C_1$-$C_4$-Alkylen oder unsubstituiertes Phenylen oder unsubstituiertes Napthylen ist.

**5.** Verbindung gemäß einem der vorstehenden Ansprüche, wobei $R^3$ an wenigstens einer von der 2- oder 4-Position bezogen auf die Bindung des Phenylrings an das Guanidin-N an den Phenylring gebunden ist.

**6.** Verbindung gemäß einem der vorstehenden Ansprüche, wobei $R^5$, $R^6$ jeweils unabhängig $C_1$-$C_4$-Alkyl sind und wobei n 2 oder 3 ist.

**7.** Verbindung gemäß einem der vorstehenden Ansprüche, die ausgewählt ist aus

- einem Salz von (E)-2-(3-Benzoyl-2-(4-methoxyphenyl)guanidino)ethylsulfonat oder der Säure davon;
- einem Salz von (E)-4-(3-Benzoyl-2-(4-methoxyphenyl)guanidino)phenylsulfonat oder der Säure davon;
- einem Salz von (E)-2-(3-Benzoyl-2-(4-methoxyphenyl)guanidino)phenylsulfonat oder der Säure davon;
- einem Salz von (E)-8-(3-Benzoyl-2-(4-methoxyphenyl)guanidino)naphthyl-2-sulfonat oder der Säure davon;
- einem Salz von (E)-2-(3-Benzoyl-2-(4-fluorphenyl)guanidino)phenylsulfonat oder der Säure davon.

**8.** Verfahren zum Herstellen einer Verbindung der allgemeinen chemischen Formel I gemäß einem der Ansprüche 1 bis 7, wobei das Verfahren Behandeln einer Thioharnstoffverbindung mit der allgemeinen chemischen Formel II:

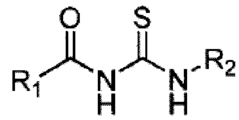

II

wobei $R^1$ und $R^2$ wie in Anspruch 1 definiert sind, mit einer primären Aminverbindung mit der allgemeinen chemischen Formel III:

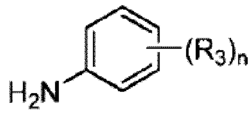

III

wobei $R^3$ und n wie in Anspruch 1 definiert sind, umfasst.

**9.** Wässrige Plattierungsbadzusammensetzung für die stromlose Abscheidung von Nickel oder einer Nickellegierung, wobei die Zusammensetzung umfasst:

(i) eine Quelle von Nickelionen und wenigstens ein Reduktionsmittel für Nickelionen, wobei die wässrige Plattierungsbadzusammensetzung ferner umfasst:
(ii) wenigstens eine Beschleunigerverbindung, die eine 1-Acylguanidinverbindung gemäß einem der Ansprüche 1 bis 7 ist.

**10.** Wässrige Plattierungsbadzusammensetzung gemäß Anspruch 9, wobei die Konzentration aller Beschleunigerverbindungen in der wässrigen Plattierungsbadzusammensetzung von 1 µmol/l bis 10.000 µmol/l beträgt, vorzugsweise von 10 µmol/l bis 1.000 µmol/l.

**11.** Wässrige Plattierungsbadzusammensetzung gemäß einem der Ansprüche 9 und 10, wobei die wässrige Plattierungsbadzusammensetzung ferner wenigstens einen Komplexbildner umfasst.

**12.** Wässrige Plattierungsbadzusammensetzung gemäß Anspruch 9 bis 11, wobei das wenigstens eine Reduktionsmittel ausgewählt ist aus der Gruppe umfassend Hypophosphitionen, Boranverbindungen, Hydrazin und Formaldehyd.

**13.** Verfahren zum stromlosen Abscheiden von Nickel oder einer Nickellegierung, wobei das Verfahren folgende Verfahrensschritte umfasst:

(A) Bereitstellen eines Substrats; und
(B) Inkontaktbringen des Substrats mit einer wässrigen Plattierungsbadzusammensetzung gemäß einem der Ansprüche 9 bis 11, so dass eine Nickel- oder Nickellegierungsbeschichtung auf dem Substrat abgeschieden wird.

**14.** Verfahren zum Beschleunigen einer wässrigen Plattierungsbadzusammensetzung für die stromlose Abscheidung von Nickel oder einer Nickellegierung, wobei das Verfahren folgende Verfahrensschritte umfasst:

(i) Bereitstellen der wässrigen Plattierungsbadzusammensetzung, die eine Quelle von Nickelionen und wenigstens ein Reduktionsmittel für Nickelionen umfasst; und
(ii) Zugeben wenigstens einer Verbindung zu der wässrigen Plattierungsbadzusammensetzung, wobei die wenigstens eine Verbindung eine 1-Acylguanidinverbindung mit der allgemeinen chemischen Formel I ist:

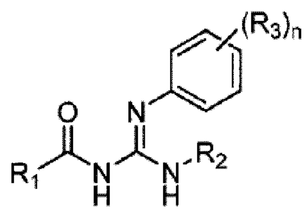

I

wobei

$R^1$ ausgewählt ist aus der Gruppe umfassend Alkyl und Aryl, wobei Alkyl unsubstituiert oder substituiert ist und Aryl substituiert oder unsubstituiert ist;
$R^2$ ein Rest mit der allgemeinen chemischen Formel $-R^4-SO_3M$ ist, wobei $R^4$ ausgewählt ist aus der Gruppe umfassend Alkylen und Arylen, wobei Alkylen unsubstituiert oder substituiert ist und Arylen unsubstituiert oder substituiert ist;
wobei M Wasserstoff oder ein Metallatom oder ein anderer kationenbildender Rest mit einer Valenz von eins ist oder ein m-ter Bruchteil eines Metallatoms oder eines anderen kationenbildenden Rests mit einer Valenz von m ist, wobei m 2 oder 3 ist;
$R^3$ ausgewählt ist aus der Gruppe umfassend -Hal, $-OR^5$ und $-NR^5R^6$, wobei $R^5$, $R^6$ Reste unabhängig ausgewählt aus der Gruppe umfassend Wasserstoff, Alkyl und Aryl sind, wobei Alkyl unsubstituiert oder substituiert ist und Aryl unsubstituiert oder substituiert ist; und
n eine ganze Zahl in dem Bereich von 1 bis 5 ist.

**Revendications**

1. Composé qui est un composé de 1-acylguanidine répondant à la formule chimique générale I :

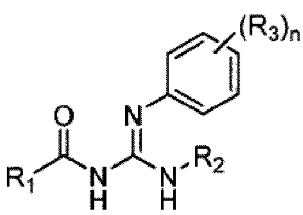

I

dans lequel

$R^1$ est choisi dans le groupe comprenant les groupes alkyle et aryle, les groupes alkyle étant non substitués ou substitués et les groupes aryle étant non substitués ou substitués ;
$R^2$ est un radical répondant à la formule chimique générale $-R^4-SO_3-M$, $R^4$ étant choisi dans le groupe comprenant les groupes alkylène et arylène, les groupes alkylène étant non substitués ou substitués et les groupes arylène étant non substitués ou substitués ; M étant l'atome d'hydrogène ou un atome métallique ou un quelconque autre radical formant un cation ayant une valence de 1 ou étant une fraction d'un $m^{ième}$ d'un atome métallique ou d'un quelconque autre radical formant un cation ayant une valence de m, m valant 2 ou 3 ;
$R^3$ est choisi dans le groupe comprenant -Hal, $-OR^5$ et $-NR^5R^6$, $R^5$ et $R^6$ étant indépendamment choisis dans le groupe comprenant l'atome d'hydrogène et les groupes alkyle et aryle, les groupes alkyle étant non substitués ou substitués et les groupes aryle étant non substitués ou substitués ; et
n est un nombre entier allant de 1 à 5.

2. Composé selon la revendication 1, dans lequel $R^1$ est un groupe alkyle en $C_1$-$C_4$ non substitué ou phényle non substitué.

3. Composé selon l'une quelconque des revendications précédentes, dans lequel $R^1$ est un groupe phényle non

substitué.

4. Composé selon l'une quelconque des revendications précédentes, dans lequel $R^4$ est un groupe alkylène en $C_1$-$C_4$ non substitué ou phénylène non substitué ou naphtylène non substitué.

5. Composé selon l'une quelconque des revendications précédentes, dans lequel $R^3$ est lié au noyau phényle en au moins l'une des positions 2 ou 4 par rapport à la liaison du noyau phényle au N de la guanidine.

6. Composé selon l'une quelconque des revendications précédentes, dans lequel $R^5$ et $R^6$ sont chacun indépendamment un groupe alkyle en $C_1$-$C_4$ et dans lequel n vaut 2 ou 3.

7. Composé selon l'une quelconque des revendications précédentes, qui est choisi entre

   - un sel de (*E*)-2-(3-benzoyl-2-(4-méthoxyphényl)guanidino)éthylsulfonate ou l'acide de celui-ci ;
   - un sel de (*E*)-4-(3-benzoyl-2-(4-méthoxyphényl)guanidino)phénylsulfonate ou l'acide de celui-ci ;
   - un sel de (*E*)-2-(3-benzoyl-2-(4-méthoxyphényl)guanidino)phénylsulfonate ou l'acide de celui-ci ;
   - un sel de (*E*)-8-(3-benzoyl-2-(4-méthoxyphényl)guanidino)naphtyl-2-sulfonate ou l'acide de celui-ci ;
   - un sel de (*E*)-2-(3-benzoyl-2-(4-fluorophényl)guanidino)phénylsulfonate ou l'acide de celui-ci.

8. Procédé de fabrication d'un composé de formule chimique générale I tel que défini dans l'une quelconque des revendications 1 à 7, ledit procédé comprenant le traitement d'un composé de thiourée répondant à la formule chimique générale II :

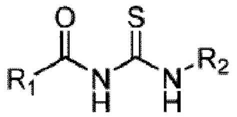

II

dans lequel $R^1$ et $R^2$ sont tels que définis dans la revendication 1,
avec un composé amine primaire répondant à la formule chimique générale III :

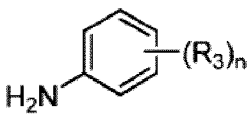

III

dans lequel $R^3$ et n sont tels que définis dans la revendication 1.

9. Composition aqueuse de bain de placage pour le dépôt autocatalytique de nickel ou d'un alliage de nickel, la composition comprenant :

   (i) une source d'ions nickel et au moins un agent réducteur pour les ions nickel,

   la composition aqueuse de bain de placage comprenant en outre

   (ii) au moins un composé accélérateur qui est un composé de 1-acylguanidine selon l'une quelconque des revendications 1 à 7.

10. Composition aqueuse de bain de placage selon la revendication 9, la concentration de tous les composés accélérateurs dans la composition aqueuse de bain de placage étant de 1 μmol/l à 10 000 μmol/l, de préférence de 10 μmol/l à 1 000 μmol/l.

11. Composition aqueuse de bain de placage selon l'une quelconque des revendications 9 et 10, la composition aqueuse de bain de placage comprenant en outre au moins un agent complexant.

**12.** Composition aqueuse de bain de placage selon la revendication 9 à 11, dans laquelle l'au moins un agent réducteur est choisi dans le groupe comprenant les ions hypophosphites, les composés boranes, l'hydrazine et le formaldéhyde.

**13.** Procédé de dépôt autocatalytique de nickel ou d'un alliage de nickel, ledit procédé comprenant les étapes de procédé suivantes :

(A) l'utilisation d'un substrat ; et
(B) la mise en contact dudit substrat avec une composition aqueuse de bain de placage selon l'une quelconque des revendications 9 à 11 pour qu'un revêtement de nickel ou d'alliage de nickel soit déposé sur ledit substrat.

**14.** Procédé d'accélération d'une composition aqueuse de bain de placage pour le dépôt autocatalytique de nickel ou d'un alliage de nickel, ledit procédé comprenant les étapes de procédé suivantes :

(i) l'utilisation de ladite composition aqueuse de bain de placage comprenant une source d'ions nickel et au moins un agent réducteur pour les ions nickel ; et
(ii) l'ajout d'au moins un composé à ladite composition aqueuse de bain de placage, ledit au moins un composé étant un composé de 1-acylguanidine répondant à la formule chimique générale I :

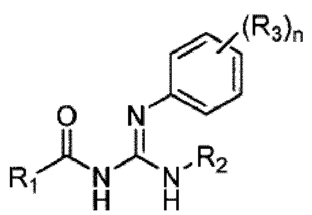

I

dans lequel

$R^1$ est choisi dans le groupe comprenant les groupes alkyle et aryle, les groupes alkyle étant non substitués ou substitués et les groupes aryle étant substitués ou non substitués ;
$R^2$ est un radical répondant à la formule chimique générale $-R^4-SO_3M$, $R^4$ étant choisi dans le groupe comprenant les groupes alkylène et arylène, les groupes alkylène étant non substitués ou substitués et les groupes arylène étant non substitués ou substitués ; M étant l'atome d'hydrogène ou un atome métallique ou un quelconque autre radical formant un cation ayant une valence de 1 ou étant une fraction d'un $m^{ième}$ d'un atome métallique ou d'un quelconque autre radical formant un cation ayant une valence de m, m valant 2 ou 3 ;
$R^3$ est choisi dans le groupe comprenant -Hal, $-OR^5$ et $-NR^5R^6$, $R^5$ et $R^6$ étant des radicaux indépendamment choisis dans le groupe comprenant l'atome d'hydrogène et les groupes alkyle et aryle, les groupes alkyle étant non substitués ou substitués et les groupes aryle étant non substitués ou substitués ; et
n est un nombre entier allant de 1 à 5.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2762723 A
- EP 0152601 A1
- EP 1281787 A
- US 2935425 A
- US 3338726 A
- US 3597266 A
- US 3915716 A
- US 4780342 A
- US 3953654 A


### Non-patent literature cited in the description


- **S.CUNHA ; B.R.DE LIMA ; A.R.DE SOUZA.** *Tetrahedron Lett.,* 2002, vol. 43, 49-52